# EUROPEAN PATENT SPECIFICATION

(11) **EP 1 664 536 B1**
(45) Date of publication and mention of the grant of the patent: **17.10.2007**
(21) Application number: 04775311.6
(22) Date of filing: 16.08.2004
(51) Int. Cl.: F04B 43/12, A61M 39/10

(54) **A HOSE CONNECTING DEVICE FOR CONNECTION TO A PERISTALTIC PUMP**
SCHLAUCHVERBINDUNGSVORRICHTUNG ZUR VERBINDUNG MIT EINER SCHLAUCHPUMPE
DISPOSITIF DE RACCORD DE MANCHON

(30) Priority: 21.08.2003 SE 0302261
(43) Date of publication of application: 07.06.2006
(73) Proprietor: Medical Vision Research & Development AB, S-131 30 Nacka (SE)
(72) Inventor: MÖLLSTAM, Anders, S-132 36 Saltsjö-Boo (SE); PETTERSSON, Ingvar, S-246 57 BARSEBÄCKS HAMN (SE)
(74) Representative: Axelsson, Nils Ake A.L.
(86) International application number: PCT/SE2004/001198
(87) International publication number: WO 2005/019648

(56) References cited:
- EP-A1- 1 048 848
- EP-A2- 0 579 916
- DE-U1- 8 406 203
- US-A- 4 184 815
- US-A- 4 976 590
- US-A- 4 997 421
- US-A- 5 131 823
- US-A- 5 192 273
- US-B1- 6 494 864

## Description

### Technical field

The present invention relates to the medical field and more particularly to a hose connecting device as specified in the preamble of claim 1. Such a device is known eg from US-A-5 131 823.

### Background of the invention

This known device comprises a support which includes a seating that is open on one side for receiving a coupling segment connected to the hose. The coupling segment has fixedly connected thereto an axial abutment element that is pressed against a supportive surface on said support, wherewith the abutment element defines an acute angle with the direction of the tension force exerted by the pump on the coupling element. This tension force is the result of a tension force exerted by the pump on the hose and/or by virtue of the hose being elastically extensible.

This known device is thus based on the exertion of some form of tension force on the hose and therewith also on the segment to which the abutment element is fixed. The abutment element is pressed against said supportive surface as a result of this tension force. Thus, this tension force is necessary in order for the coupling to function satisfactorily. There is a danger of the coupling element loosening from the support in the absence of said tension force, therewith causing the hose to loosen from the pump.

### Object of the invention

One object of the present invention is to provide an improved hose connecting device that is independent of the forces generated by the pump or by the elasticity of the hose. The coupling shall retain the hose connected to the pump in an unchanged position even though the pump should stop or the hose be subjected to an elastic change in shape. The hose shall, thus, not loosen spontaneously during use.

Another object is to provide a connecting device that can be readily manoeuvred manually both when mutually connecting and separating the coupling components.

Yet another object of the invention is to provide a connecting device that can co-act with a plurality of existing pump connections without needing to make expensive modifications thereto.

### Summary of the invention

These objects are fulfilled by the present invention set forth in the independent claims. Suitable embodiments of the invention will be evident from the dependent claims.

The fastening techniques described in the exemplifying devices are achieved with touch and clothes fasteners, magnetic fasteners, self-adhesive glue tape, and shape locking techniques, such as snap acting, clamp acting or wedge acting techniques.

### A brief description of the drawings

The present invention will now be described in more detail with reference to exemplifying embodiments thereof and also with reference to the accompanying drawings, in which
- Figure 1: is a plan view of a not covered by the present invention, but contained for illustrative purposes hose connecting device
- Figure 2: is a plan view of a hose connecting device not covered by the present invention, but contained for illustrative purposes;
- Figure 3: is a perspective view of a hose connecting device not covered by the present invention, but contained for illustrative purposes fitted to a hose;
- Figure 4: is a perspective view of a support element not covered by the present invention, but contained for illustrative purposes;
- Figure. 5: is a perspective view of a hose connecting device not covered by the present invention, but contained for illustrative purposes.
- Figure 6: is a perspective view of a hose connecting device not covered by the present invention, but contained for illustrative purposes.
- Figure 7: is a perspective view of a hose connecting device not covered by the present invention, but contained for illustrative purposes.
- Figure 8: is perspective view of a hose connecting device not covered by the present invention, but contained for illustrative purposes.
- Figure 9: is perspective view of an embodiment according to the invention; and
- Figure 10: is a perspective view of a hose connecting device not covered by the present invention, but contained for illustrative purposes.

### Description of the invention

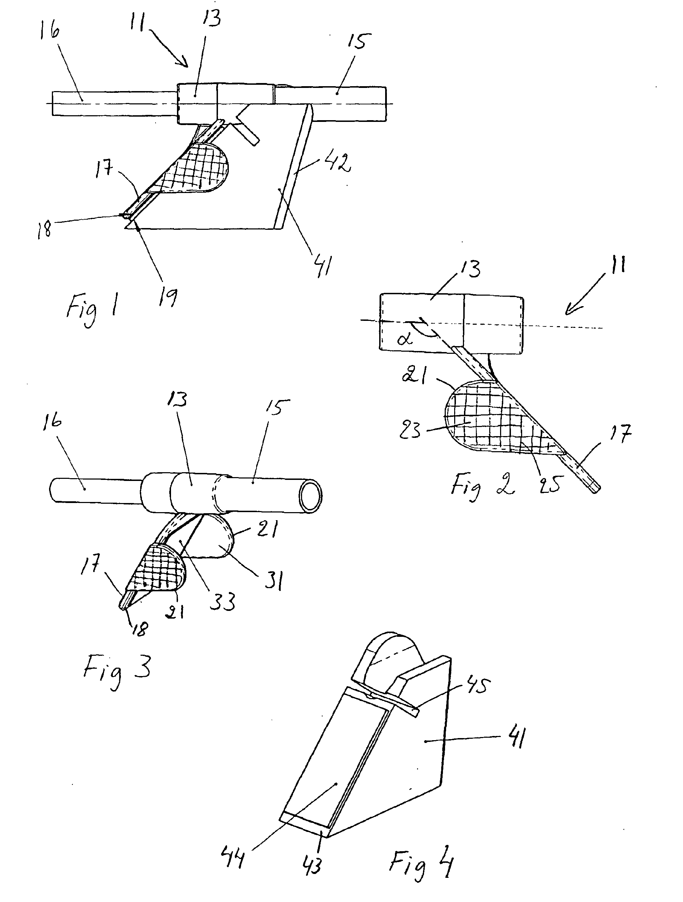

Figure 1 illustrates a hose connecting device 11 that includes a carrier 13 which is firmly connected to an infusion hose 15. The carrier 13 is preferably in the form of a tubular element that has at one end a first internal diameter for attachment to the infusion hose and at its other end a second internal diameter for attachment to a supply hose 16. The carrier 13 also includes an angled part 17 that has an abutment surface 18 on one side thereof. The abutment surface 18 coacts with a coupling surface 19 on a supportive part 41. In turn, the supportive part is fixed to a peristaltic pump (not shown) in a conventional manner, via mounting means 42. Figure 1 thus shows the state of the hose connecting device when coupled to the supportive part.

Figure 2 illustrates solely the hose connecting device 11 with the angled part 17 firmly connected to the carrier 13, said angled part defining an angled α with the carrier sign 13 wherein 180° ≥ α ≥ 90°. Although the illustrated angled part 17 is fixed to the carrier 13, said part may, alternatively be hinged to the carrier for angular movement within said angular range. The angled part 17 also includes gripping means 21 which extend at right angles outwardly from the angled part 17. The outwardly facing gripping surfaces 23 of the gripping means 21 are provided with friction enhancing means 25, for instance in the form of serrations.

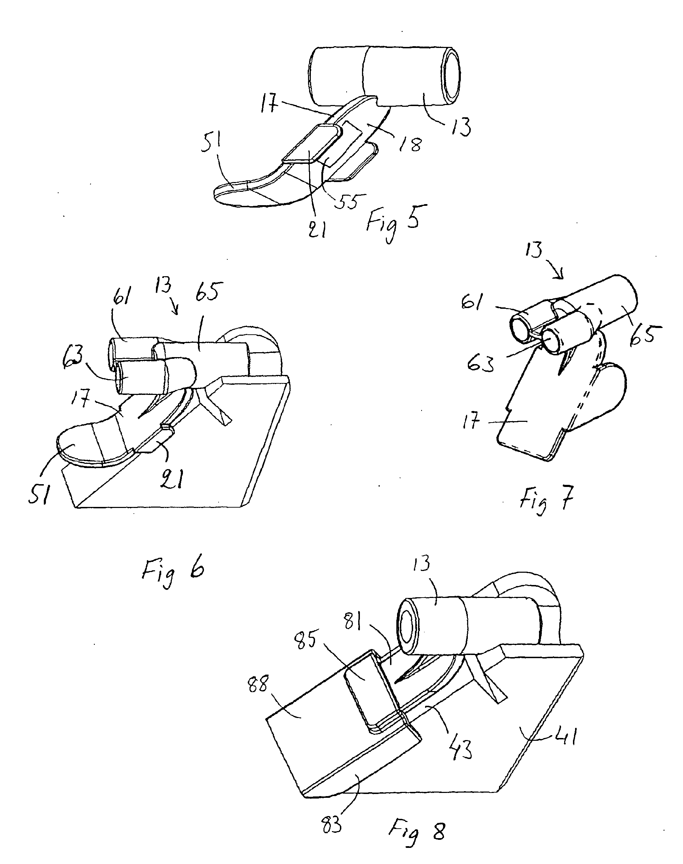

Figure 3 illustrates the carrier 13 with the fusion hose 15 and the supply hose 16 connected thereto. The figure also shows that the gripping means 21 connected to the angled part 17 include inwardly facing guide surfaces 31 that extend at right angles to the abutment surface 18. The abutment surface 18 of the angled part 17 is provided with a first coupling element 33.

As shown in figure 4, the supportive part 41 includes a coupling surface 43 which, in turn, is provided with a second coupling element 44. In this respect, the first coupling element 33 on the abutment surface 18 is intended for co-action with the second coupling element 44 on said coupling surface such as to provide a coupling which will function to prevent relative movement between the coupling elements 33, 44 in a direction parallel with the coupling surface 43. On the other hand, inactivation of the coupling is permitted at a force which is at least equal to the tension force generated by the pump in said hose in a direction that has a mutual separating effect on the abutment surface and the coupling surface. Compare the assembled connected device 11 according to figure 1.

Because the supportive part 41 illustrated in the figures is a conventional device, the device also includes a slot 45 whose use originates from earlier coupling methods and which is preferably blocked to avoid cleaning problems.

Figure 5 illustrates a hose connecting device in which the angled part 17 of the carrier 13 is provided with a gripping portion 51 in the form of an end piece protruding out from the angled part 17. This gripping portion enables the coupling to be readily released by drawing the abutment surface away from the coupling surface. In this device, the abutment surface 18 also includes a first coupling element 55. As will be evident from the figure, the gripping means 21 connected to the angled part 17 have smaller surfaces than the gripping means 21 of the first hose connecting device of figures 1-4.

Figure 6 illustrates a hose connecting device in which the carrier 13 includes two supply connections 61, 63 which merge with a pump connection 65. The angled part 17 of this hose connection device has the form shown in figure 5 in other respects, i.e. it includes a corresponding gripping part 51 and gripping means 21.

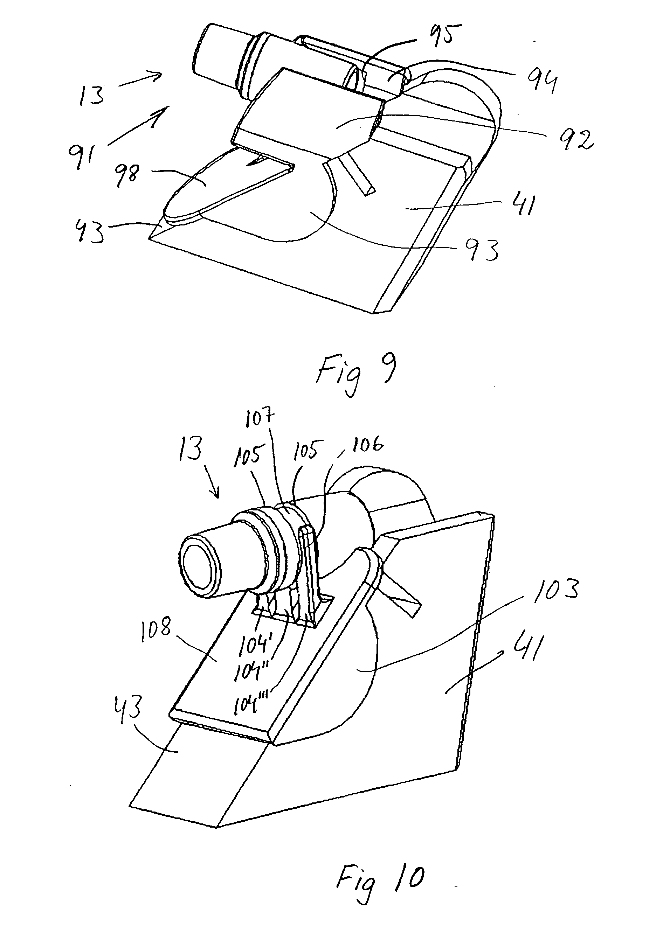

Figure 7 illustrates a hose connecting device which, similar to the carrier 13 of the figure 6 device, includes two supply connections 61, 63 which merge with a pump connection 65. However, the angled part 17 of the figure 7 device has the form of the angled part shown in figures 1-3.

The first and the second coupling devices shown in figures 1-7 are comprised of touch and close fasteners or of glue/tape applied to the coupling surface. Alternatively, the first and second coupling devices may comprise a magnet placed on either the abutment surface or on the coupling surface.

Figure 8 illustrates a hose connecting device in which the carrier 13 includes a modified angled part 81, which also includes an abutment surface corresponding to that earlier described. In the case of the figure 8 device, the gripping means described with respect to the earlier devices function as guide surfaces 83 for centring an adapter sleeve 88 when mounting said sleeve to the supportive part 41. The adapter sleeve 88 includes a first coupling device 85 in the form of a pocket which defines, together with supportive part 41, a slot into which the angled part 81 can be pressed and retained so as to fixate the carrier to the coupling surface 43 of the supportive part 41. The guide surfaces 83 and the coupling device 85 are both arranged in the adapter sleeve 88, which is a permanent fixture on the supportive part 41. Thus, this device provides a hose coupling having a negative radial projection that is pushed down into a slot on the fixated adapter, wherewith the angle between carrier 13 and abutment surface 43 is α, where 180° ≥ α ≥ 90°.

Figure 9 illustrates an embodiment of a hose connecting device 91 according to the invention. The device is intended to be fitted to the coupling surface 43 of an existing supportive part 41 in accordance with each of the earlier described and illustrated devices, although fixedly mounted in the case of this embodiment. The device comprises a carrier 13 to which the hose (not shown) is connected, and an adapter sleeve 98 which is fixedly mounted to the supportive part 41. As will be seen from the figure, the adapter sleeve includes guide surfaces 93 and a cylindrical abutment surface 94 in a coupling part 92, into which the carrier 13 can be pressed and therewith fastened. In this case, the cylindrical fitting forms a clamping joint and may be provided internally with axial support means 95 in the form of an abutment surface against which the carrier abuts the bottom when fitted.

Figure 10 illustrates a hose-connecting device that is intended to be fitted to the coupling surface 43 of an existing supportive part 41. Correspondingly to what has been described above, a carrier 13 is intended for connection to an adapter sleeve 108 that includes guide surfaces 103 for centred fixed mounting of the sleeve to the supportive part 41. The adapter sleeve 108 includes two or more outwardly projecting corners, three such corners 104'. 104", 104'" in the case of the illustrated device, which are formed to abut the barrel surface of the carrier and/or engage with grooves 107 that include ring-shaped collars 105 formed in the carrier. The outer corners 104'. 104"' are provided with curved surfaces 106 which are complementary to the curvature of the carrier barrel surface and which partially resiliently embrace the carrier 13 to provide a snap-connecting effect. In the case of the figure 10 embodiment, the abutment surface is formed between a collar 105 and one or more corners 104', 104", 104"'. Thus, this embodiment constitutes an example of an angle α between the carrier 13 and the abutment surface, where α = 90°.

## Claims

1. A hose connecting device (91) comprising a carrier (13) which is fixedly connected to an infusion hose (15) and which carrier (13) is attached to a supportive part (41) fixedly connected to a peristaltic pump, **characterized in that**, the connecting device (91) includes a coupling part (92) located on an adapter sleeve (98) said adapter sleeve (98) having the form of an angled part with respect to the axis of the coupling part (92) said angled part being provided with a first coupling element (33) cooperating with a second coupling element (44) on a coupling surface (43) affixed onto the supportive part (41) for providing a fixed connection between the adapter sleeve (98) and the supportive part (41), wherein the coupling part (92) includes a cylindrical abutment surface (94) located in the coupling element (92) and intended for co-action with the carrier (13) so as to form a clamping joint.

2. A hose connecting device according to claim 1, **characterized in that** the cylindrical abutment surface (94) is provided internally with axial support means (95).

3. A hose connecting device according to any one of claims 1-2,
**characterized in that** the angle between the axial direction of the carrier and the direction of the abutment surface (94) or a supportive surface in the supportive element (95) co-acting with the carrier lies in the range between 90° and 180°

## Patentansprüche

1. Schlauchverbindungsvorrichtung (91), welche einen Träger (13) aufweist, der fest mit einem Infusionsschlauch (15) verbunden ist, wobei der Träger (13) an einem tragenden Abschnitt (41) befestigt ist, welcher fest mit einer Peristaltikpumpe verbunden ist, **dadurch gekennzeichnet, dass** die Verbindungsvorrichtung (91) einen Kopplungsabschnitt (92) aufweist, der auf einer Anschlusshülse (98) vorhanden ist, wobei die Anschlusshülse (98) die Form eines gewinkelten Teils bezüglich der Achse des Kopplungsabschnitts (92) hat, wobei der gewinkelte Teil mit einem ersten Kopplungselement (33) ausgestattet ist, welches mit einem zweiten Kopplungselement (44) auf einer Kopplungsfläche (43) zusammenwirkt, wobei die Kopplungsfläche (43) zur Bereitstellung einer festen Verbindung zwischen der Anschlusshülse (98) und dem tragenden Abschnitt (41) auf dem tragenden Abschnitt (41) befestigt ist, wobei der Kopplungsabschnitt (92) eine zylindrische Stützfläche (94) aufweist, die in dem Kopplungselement (92) vorhanden und zur Zusammenwirkung mit dem Träger (13) zur Bildung einer Klemmverbindung bestimmt ist.

2. Schlauchverbindungsvorrichtung nach Anspruch 1, **dadurch gekennzeichnet, dass** die zylindrische Stützfläche (94) innen mit axialen tragenden Vorrichtungen (95) ausgestattet ist.

3. Schlauchverbindungsvorrichtung nach einem der Ansprüche 1 oder 2, **dadurch gekennzeichnet, dass** der Winkel zwischen der axialen Richtung des Trägers und der Richtung der Stützfläche (94) oder einer tragenden Fläche in dem mit dem Träger zusammenwirkenden tragenden Element (95) im Bereich zwischen 90° und 180° liegt.

## Revendications

1. Dispositif de raccordement de tuyaux (91) comprenant un élément porteur (13) fixé sur une tubulure de perfusion (15), élément porteur (13) qui est monté sur une partie de support (41) montée fixement sur une pompe péristaltique, **caractérisé en ce que** le dispositif de raccordement (91) comprend une partie de couplage (92) située sur un manchon (98), ce manchon (98) présentant la forme d'une partie qui forme un angle par rapport à l'axe de la partie de couplage (92), la partie formant un angle étant équipée d'un premier élément de couplage (33) coopérant avec un deuxième élément de couplage (44) sur une surface de couplage (43) fixée sur la partie de support (41) afin d'assurer une liaison fixe entre le manchon (98) et la partie de support (41), la partie de couplage (92) comprenant une surface de butée cylindrique (94) située dans l'élément de couplage (92) et destinée à coopérer avec l'élément porteur (13) afin de constituer une jonction par serrage.

2. Dispositif de raccordement de tuyaux selon la revendication 1, **caractérisé en ce que** la surface de butée cylindrique (94) est pourvue, à l'intérieur, de moyens de support axiaux (95).

3. Dispositif de raccordement de tuyaux selon l'une quelconque des revendications 1-2, **caractérisé en ce que** l'angle entre la direction axiale de l'élément porteur et la direction de la surface de butée (94) ou d'une surface de support présente sur le moyen de support (95) qui coopère avec l'élément porteur, est compris entre 90° et 180°.
